# EUROPEAN PATENT APPLICATION

(11) **EP 3 695 881 A1**
(43) Date of publication of application: **19.08.2020**
(21) Application number: 19157614.9
(22) Date of filing: 18.02.2019
(51) Int. Cl.: A61N 5/06, A61F 7/00, A45D 2/36, A61N 1/20, A61N 1/32, A61N 1/04, A45D 2/08

(54) **ROLLER DEVICE FOR TREATING HAIR WITH MICRO-CURRENTS**

(71) Applicant: L'OREAL, 75008 Paris (FR)
(72) Inventor: PLANARD-LUONG, Thi Hong Lien, BP 553 94152 Chevilly Larue (FR)

(57) **Abstract**

The invention relates to an apparatus for treating hair, comprising a deformable roller (7) configured for being sleeved around a strand of hair when the apparatus is in the operating configuration, that the apparatus comprises at least one energy diffuser connected to an energy generator.

## Description

### Field of the invention

The present invention relates to a method for treating at least one lock of hair and to a device for performing such a method.

Nowadays several concerns are still not addressed regarding hair treatments. Current hair treatments such as oxidation coloration, hair shaping (curl, straightening), permanent process or bleaching may damage hair to some extent due to very oxidative ingredients or high temperature. Hair care solution most often only brings temporary and surface only perceived repair.

### Background of the invention

In WO15027190, an apparatus for treating hair fiber using direct micro-currents is disclosed. The apparatus comprises electrodes including rows of teeth of different polarity and flanked by guard combs made of an insulating material.

US9038645 discloses an apparatus for subjecting strands of hair to a dielectric plasma treatment connected to a high voltage source.

The invention aims to further improve apparatuses for electrical treatments of the hair with micro-currents to add performance in terms of lastingness and security inter alia.

### Summary of the invention

According to a first of its aspects, the invention provides an apparatus for treating hair, comprising a deformable roller configured for being sleeved around a strand of hair when the apparatus is in the operating configuration and the apparatus comprises at least one energy diffuser connected to an energy generator.

The invention allows easier application of micro-currents for treating hair fibers. The treatment is performed homogenously on the fibers of the strand of hair. This improves penetration of ingredients or grafting of molecules at the hair surface. The invention is particularly useful for performing hair shaping, in particular permanent hair shaping, and/or hair care.

According to its second aspect, the invention relates to a method for treating hair using an apparatus according to any one of the preceding claims, comprising exposing a strand of hair around which the roller is sleeved to energy diffuser.

Preferably, the method comprises applying a composition to the strand of hair to be treated and exposing the strand of hair to micro-currents by keeping the roller static.

The invention may enable to reduce the time of diffusion of the ingredients in the hair fiber, for example by a factor of 5. In addition, the ingredients have a longer lasting within the hair fiber and are removed more slowly from hair.

The curls remain longer and the curling process is done less frequently. The styling is improved, with or without applying a composition on the hair.

The invention is also particularly useful for improvement of penetration into the hair cortex of hair care or curling or styling ingredients. For instance, hydrophobic ingredients, shiny ingredients, UV protection ingredients. The perceived effect is improved and remains longer, for example after more than 3 shampoos.

The first and second electrodes on respective sides of the apparatus may be spaced apart from each other, preferably by a distance of between 1 and 10 cm, more preferably of about 5 cm.

The apparatus may be configured to heat the hair independently of the application of the micro-currents. For example, the apparatus may be configured to heat the hair, before, during and/or after the application of the micro-currents. The allows to improve the effect of the treatment by micro-currents.

The apparatus may comprise a sensor for measuring the temperature of the hair during exposure to the micro-currents. The apparatus may further comprise an alarm system that gives a visual or audio signal when the temperature of the hair exceeds a pre-determined value, to avoid damage the hair fibers during treatment.

The apparatus may comprise different first and second electrodes for respectively applying micro-currents of different types, for example a pulsed voltage and a non-pulsed voltage. For example, the contact surfaces may be the first and second electrodes configured for applying a pulsed micro-current and the teeth may be the first and second electrodes configured for applying a non-pulsed current.

The apparatus may comprise a selector for switching from the application of one type of micro-current to another, by connecting respectively the corresponding electrodes to the voltage generator. The selection may be automatic, the mounting of a pair of electrodes on the apparatus controlling a mode of operation of the apparatus and the corresponding voltage.

The non-pulsed current may be a DC current.

By "DC microcurrent" it is meant a microcurrent that does not change polarity, i.e. unidirectional. The DC voltage is preferably continuous. Preferably, the DC voltage ranges between 70 V and 130 V, more preferably between 80 V and 120 V. The intensity is preferably limited, so that the user is not exposed to a risk of electrical shock.

By "continuous", it should be understood that the voltage does not have interruptions, i.e., remain zero during a non-zero period, contrary to a "pulsed" voltage which may be interrupted, i.e., remain zero during a non-zero period, for example periodically.

In a variant, the non-pulsed current is continuous and may change polarity, preferably at constant intervals, for example every 1 to 5 seconds.

The voltage of the electrical pulses of the pulsed micro-current may be high enough to electroporate the hair fibre during the treatment, for example of a peak voltage of between 100 V and 700 V. The polarity of the electrical pulses preferably changes between two consecutive pulses.

By "electroporation", one should understand a method that increases permeability of the hair fibre to the penetration of some active ingredients.

In the present invention, the voltage is measured between the at least one first and at least one second electrodes that serve to apply the voltage to the hair.

The apparatus may comprise a user interface, for example a screen, preferably a tactile screen, for selecting the treatment and enter parameters thereof. Treatment parameters may include the type of current to be applied, the voltage and duration thereof, the duration of operation of the apparatus. The values may be pre-set and by selection of a corresponding treatment program, the values corresponding to the selected treatment are automatically loaded. The selection may also be the result of the identification of a type of electrodes mounted on the apparatus.

At least some of the treatment parameters may be displayed on the screen during the treatment. The apparatus may also be configured to allow the user to adjust the corresponding parameters by using tactile screen with the help of extra buttons, if necessary.

The apparatus may be configured to allow the user to indicate, before the start of the treatment, whether different types of micro-current should be applied in a single session, i.e. the micro-currents of different types are applied consecutively, preferably being separated by no more than 1 hour and for example without washing the hair between application of different types of micro-current. In such case, the apparatus may be configured to automatically switch between application of a different type of micro-currents when application of one type of micro-currents is completed.

The invention also provides according to another of its aspects a kit for treating at least one lock of hair, the kit comprising an apparatus according to the invention and at least one container comprising the composition to be applied to the hair. The kit may comprise a packaging in which the apparatus and the at least one container are packaged.

The composition may be applied on hair before and/or during exposure to the micro-current.

The duration of application of the micro-currents may be chosen according to the desired effect. The duration of exposure to electrical pulses is preferably between 1 and 5 min, preferably about 2 min. The duration of exposure to non-pulsed micro-current is preferably between 3 and 7 min, for example about 5 min.

Preferably, the temperature of the hair is less than 80°C during exposure.

The treatment may comprise heating the hair for crosslinking an hydrophobic, UV protecting or smoothening coating on the hair cuticle during the treatment, after application of the micro-currents.

### Preferred embodiments

According to further advantageous aspects of the invention, the device comprises one or several of the following features taken in isolation or in any technically possible combinations:
- The energy diffuser comprises at least two electrodes connected to at least one of a current generator or a voltage generator. This is a first embodiment of the invention, using currents.
- The energy diffuser is a thermic diffuser connected to at least one of a thermic or an electrical generator. This is a second embodiment of the invention, using heat.
- The energy diffuser is an array of light emitting diodes connected to an electric generator, notably a LED or an OLED or a LASER diode. In that case, the energy diffuser is easy to manufacture.
- The energy diffuser is a light diffuser, notably an optical fiber connected to a light generator. This is a third embodiment of the invention, using light.
- The energy diffuser comprised two pieces facing one to another, on each lateral face of the roller. The current flow is accurate.
- The energy diffuser comprises a single piece disposed along the roller. The cost of manufacturing is lowered.
- It comprises at least two rollers connected to the same energy generator. The realization of the circuit is simple.
- The roller is configured for being automatically sleeved around a strand of hair and automatically unsleeved when a stress is applied along the longitudinal axis of the roller. It is easy to manipulate.
- The roller is configured to define two physical stable shapes: a sleeved position and an unsleeved position. These positions are predetermined for a better use.
- Each of the diffuser extends from one first axial end of roller towards a second axial end of the roller when observed along a longitudinal direction X of the roller in the rest configuration. The position is clear to retrieve after twisting.
- Each roller has the shape of a flattened tube in the rest configuration, the diffuser extending on each of the lateral sides of the tube. It is easy to put away and to tudy.
- The roller has the shape of a spiral, a screw, a scroll or a spring in the operating configuration.
- The roller defines an internal space for accommodating the strand of hair in the operating configuration.
- The roller comprises on an internal face, a contact surface for coming into contact with the strand of hair in an operating configuration of the apparatus. It contacts the hair for improved results.
- It comprises a controller to control the energy delivered by the generator to the energy diffuser.
- The voltage generator is configured for applying a pulsed biphasic current on the electrodes, a non-pulsed continuous current which changes polarity, preferably every 1 to 5 seconds.

### Detailed description

The invention will be understood better from reading the following detailed description of non-limiting exemplary embodiments thereof and from studying the appended drawing, in which:
Fig. 1 is a perspective views of an embodiment of an apparatus according to the invention, in an operative configuration,
Fig. 2 and Fig. 3 are perspective views of an embodiment of an apparatus according to figure 1, in first rest configuration,
Fig. 4A to 4C show another embodiment of an apparatus according to the invention, and
Fig.5 is a schematic illustration of an example of an apparatus of the present invention.

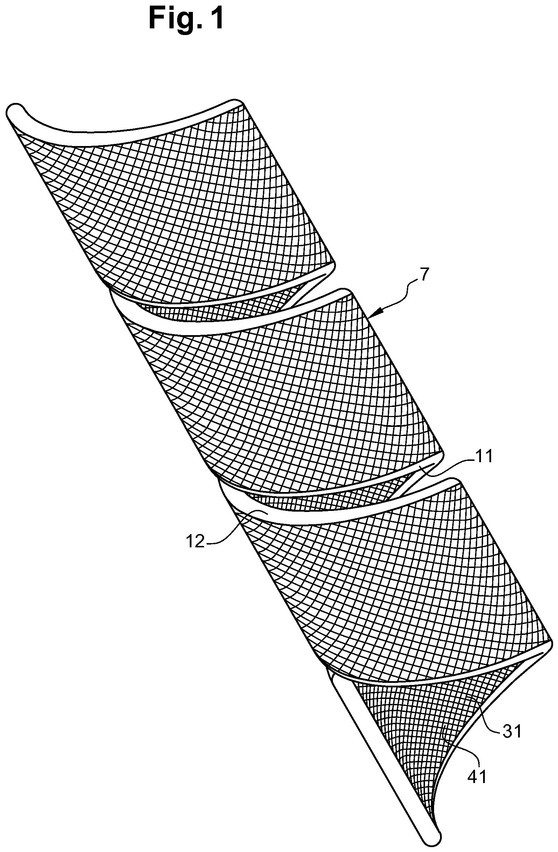

In the embodiment illustrated in Figures 1, 2 and 3, the apparatus comprises a roller 7 for grasping inside a strand of hair (not shown) to be treated. The roller 7 may comprise two electrodes 11 and 12.

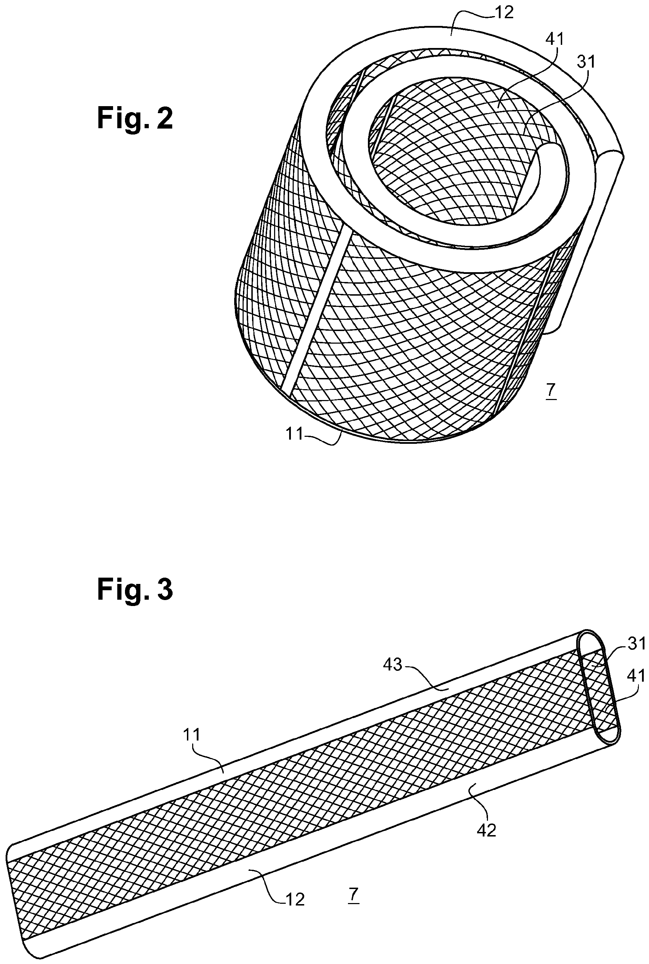

The roller 7 may comprise, on an internal face 31 thereof, a contact surface 41 for coming into contact with the strand of hair in an operating configuration of the apparatus (not shown). In the illustrated embodiment, the lateral faces 42, 43 of the roller of Figure 3 form respectively a first electrode 11, for example a cathode, and a second electrode 12, for example an anode, for applying a micro-current to the hair, for example a pulsed micro-current. The electrodes 11, 12 are for example connected to a voltage generator powered by a battery or by an external energy source. The voltage generator (not shown) may be housed separately. A user interface might be also provided, for example.

The electrodes 11, 12 might be flexible. They can be made of metal. The electrodes might be positioned on an insulator, which might be covered by an insulating grid. The insulator might be malleable and plastically deformable.

The apparatus may comprise actuated elements for example in the form of switches such as a push buttons. They may be connected in series with the generator and a corresponding electrode. When the apparatus comes to its operating configuration, the push buttons are pushed in and connect the electrodes to the voltage generator, thus allowing the electrodes to generate an electrical field that might be substantially transverse to the strand of hair. In a variant, the actuation elements are replaced by at least one magnetic switch.

Fig.1 shows the roller in an operative configuration, the strand of hair being inside the propeller obtained by twisting the roller 7 around the strand of hair.

Fig. 2 shows the roller in a rest configuration, totally folded and enrolled.

Fig. 3 shows the roller in another rest configuration, totally unrolled.

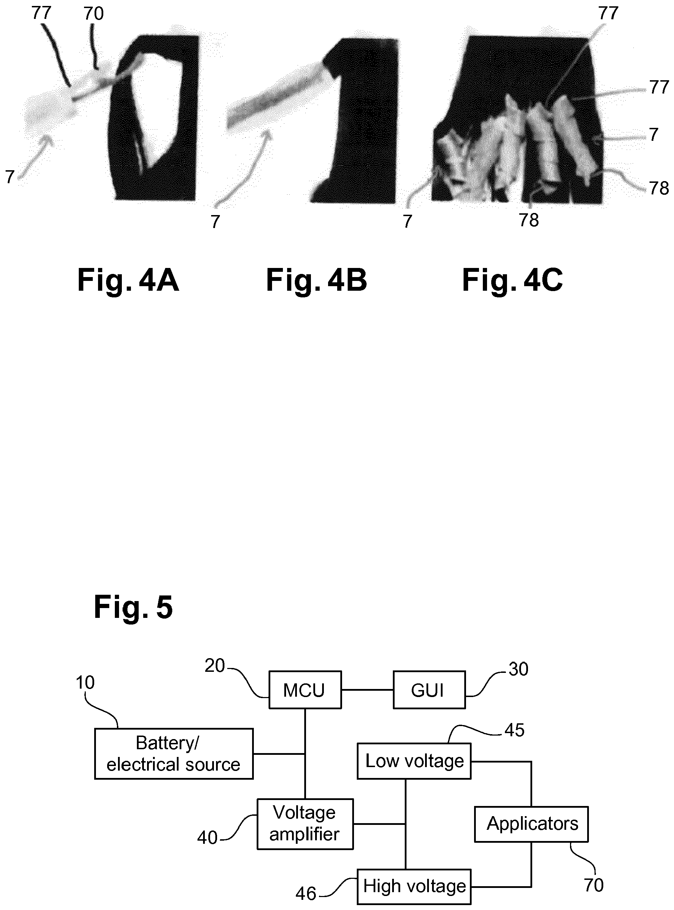

In the embodiment illustrated in Figures 4A to 4C, the apparatus comprises a deformable curler 7 for performing permanent wave treatment. The curler 7 has a shape memory and takes the shape of a spiral tube at rest. It may also take the shape of a straight tube when extended, to allow introduction therein of a lock of hair.

In order to use the apparatus, after application of a composition for permanent shaping on the strand of hair to be treated, and optionally rinsing and drying, the latter is introduced inside the tube at one end 77 thereof, with the help of a gripper 70 configured to slide inside the tube, as shown in Fig.4A. The curler 7 is then positioned on the region of the strand of hair to be treated, as shown in Fig.4B, with the tips of the hair fibers exceeding the curler 7 at another end 78. Afterwards, the curler 7 takes the spiral shape as shown in Fig.4C, before exposure to micro-currents.

The shape and size of the curler 7, for example its length, can be chosen as a function of the desired effect.

In the embodiment of Fig. 4A-4C, application of the micro-currents are performed when the hair is rolled, to change the shape of the hair and obtain permanent shaping.

Figure 5 shows components of a treatment apparatus in accordance with the invention.

The apparatus comprises one or more applicator parts 70 comprising one or more contact surfaces and the electrodes. The electrodes are connected respectively to a high voltage terminal 46 and a low voltage 45 terminal of a voltage amplifier 40.

The apparatus further comprises a microcontroller unit (MCU) 20 that controls the voltage amplifier 40. The microcontroller unit 20 may interact with a user interface 30, for example a graphical user interface (GUI).

The microcontroller unit 20 and the voltage amplifier 40 are powered by an energy source, for example a battery or a power network.

The microcontroller unit 20 controls the voltage amplifier 40 so that the voltage present at the electrodes is in accordance with the treatment to be performed, such as comprising exposure to a pulsed micro-current and/or a non-pulsed DC micro-current.

The invention is not limited to the embodiments described above.

For example, the apparatus may be configured for delivering a composition to be applied to the hair, for example before or during the treatment.

## Claims

1. An apparatus for treating hair, comprising a deformable roller (7) configured for being sleeved around a strand of hair when the apparatus is in the operating configuration, **characterized by** the fact that the apparatus comprises at least one energy diffuser connected to an energy generator.

2. An apparatus according to claim 1, **characterized in that** the energy diffuser comprises at least two electrodes (11, 12) connected to at least one of a current generator or a voltage generator.

3. An apparatus according to claim 1, **characterized in that** the energy diffuser is a thermic diffuser connected to at least one of a thermic or an electrical generator.

4. An apparatus according to claim 1, **characterized in that** the energy diffuser is an array of light emitting diodes connected to an electric generator, notably a LED or an OLED or a LASER diode.

5. An apparatus according to claim 1, **characterized in that** the energy diffuser is a light diffuser, notably an optical fiber connected to a light generator.

6. An apparatus according to anyone of the preceding claims, **characterized in that** the energy diffuser comprised two pieces facing one to another, on each lateral face of the roller (7).

7. An apparatus according to anyone of the claims 1 to 5, **characterized in that** the energy diffuser comprises a single piece disposed along the roller (7).

8. An apparatus according to anyone of the preceding claims, **characterized in that** it comprises at least two rollers (7) connected to the same energy generator.

9. An apparatus according to anyone of the preceding claims, **characterized in that** the roller (7) is configured for being automatically sleeved around a strand of hair and automatically unsleeved when a stress is applied along the longitudinal axis of the roller (7).

10. An apparatus according to anyone of the preceding claims, **characterized in that** the roller (7) is configured to define two physical stable shapes: a sleeved position and an unsleeved position.

11. An apparatus according to claim 1, **characterized in that** each of the diffuser extends from one first axial end of roller (7) towards a second axial end of the roller (7) when observed along a longitudinal direction (X) of the roller in the rest configuration.

12. The apparatus of any one of the preceding claims, **characterized in that** the roller (7) has the shape of a flattened tube in the rest configuration, the diffuser extending on each of the lateral sides of the tube.

13. The apparatus of any one of the preceding claims, **characterized in that** the roller (7) has the shape of a spiral, a screw, a scroll or a spring in the operating configuration.

14. The apparatus of any one of the preceding claims, **characterized in that** the roller (7) defines an internal space for accommodating the strand of hair in the operating configuration.

15. The apparatus of any one of the preceding claims, **characterized in that** the roller (7) comprises on an internal face (31), a contact surface (41) for coming into contact with the strand of hair in an operating configuration of the apparatus.

16. The apparatus of any one of the preceding claims, comprising a controller (20) to control the energy delivered by the generator (10) to the energy diffuser.

17. The apparatus of any one of the preceding claims, the voltage generator (10) being configured for applying a pulsed biphasic current on the electrodes (11, 12), a non-pulsed continuous current which changes polarity, preferably every 1 to 5 seconds.

18. A method for treating hair using an apparatus according to any one of the preceding claims, comprising exposing a strand of hair around which the roller (7) is sleeved to energy diffuser.

19. A method for treating hair according to claim 18, the method comprising applying a composition to the strand of hair to be treated and exposing the strand of hair to micro-currents by keeping the roller (7) static.
